# EUROPEAN PATENT APPLICATION

(11) **EP 4 151 918 A1**
(43) Date of publication of application: **22.03.2023**
(21) Application number: 21804158.0
(22) Date of filing: 13.05.2021
(51) Int. Cl.: F24F 8/22, A61L 9/20

(54) **AIR PURIFICATION DEVICE**

(30) Priority: 14.05.2020 ES 202030914 U
(71) Applicant: Urban Air Purifier, S.L., 08006 Barcelona (ES)
(72) Inventor: CUSÍ NAVARRO, Joaquín, 08006 Barcelona (ES); QUINTANA BARTUAL, Alberto, 08006 Barcelona (ES)
(74) Representative: Ungria López, Javier
(86) International application number: PCT/ES2021/070339
(87) International publication number: WO 2021/229130

(57) **Abstract**

Disclosed in an air purification device comprising a case that contains at least a first filtering system (7) including an M6 filter according to standard EN1822, a second filtering system (8) including UV-C lamps (11), and a third filtering system (9), wherein the second filtering system (8) is situated between the first filtering system (7) and the third filtering system (9), the UV-C light of the lamps (11) sterilising the air flow that passes through the second filtering system (8), and wherein the UV-C light of the lamps (11) has a powerful destructive effect on the DNA chains of microorganisms or cells retained in the first filtering system (7) and the third filtering system (9) and also on those of microorganisms or cells in the space delimited between the first filtering system (7) and the third filtering system (9).

## Description

### Object of the invention

The present invention relates to an air purification device to be installed in areas with pedestrian traffic in order to combat and neutralise environments that may be infected by viruses, bacteria, and other pathogens, particularly "Covid 19".

The invention is an improvement of the device described in patent WO 2020/058547 A1 consisting of a reconfiguration of the filter system and the inclusion of a specific UV system; all this to obtain a better performance in neutralising microorganisms, such as bacteria and viruses, including those of the coronavirus type.

It should be noted that the size of coronaviruses varies between 150 nanometers and 190 nanometers, such that this size is equivalent to 0.15-0.19 microns. Therefore, the invention relates to the incorporation of a series of filters capable of retaining particles/pathogens of this size in the device.

### Technical problem to be resolved and background of the invention

In recent years, due to the increasing concentration of pollutants in urban and industrial centres, people's concern for breathing clean air has increased, so that increasingly restrictive regulations have been implemented, aimed at limiting the generation of these polluting elements.

These regulations imply the necessary investment in systems that prevent pollution at ever lower levels, by the players involved in the development of those activities carried out in industrial centres and cities.

In addition to carrying out actions aimed at limiting the pollution generated, either through restrictive regulations, or by implementing processes that generate less pollution, different types of technologies have also been developed, aimed at reducing the existing pollution in the environment in an active way, capturing the air, which can be polluted with gases or suspended particles, to return it to the environment with a lower concentration of pollutants.

Among these types of technologies air purification devices commonly called "Totems" stand out, the purpose of which is to reduce the pollution index of the environment where they are located.

At present, there is a great variety of these types of "Totems" that differ in the cleaning and purification processes carried out, as well as in the elements that make them up.

For example, the Spanish invention ES2595478, which includes the improvements of an air purification device described in the also Spanish invention ES2394411, comprises a set of elements, inserted in a hollow structure through which the air is passed with the aim of filtering the solid particles in said air, and through components, to retain the polluting gases.

In most of these air purification devices the air is passed through filters that are capable of retaining particles called PM, (Particulate Matter) followed by the size of said particles, in micrometres, so that the PM₁₀, are small solid or liquid particles of dust, ash, soot, metal, cement or pollen, dispersed in the atmosphere, and the aerodynamic diameter of which is less than 10 µm.

PM particles of this type are considered polluting elements since the presence thereof alters the natural composition of the atmosphere, whether it is caused by acts of nature or by human action, reason for which they are subject to be retained by the air cleaning devices.

The filters used to retain this type of PM can be of different types, depending on the material, purpose, and operation thereof, but they are usually classified into categories based on the efficiency for retaining particles of different diameters that they are capable of blocking.

In this way, the filters classified according to the European standard EN 779 of filtration class from G1 to G4, for coarse dust, and those of filtration class M5, M6, F7, F8 and F9, for medium and fine particles, stand out. This standard imposes an average filtration efficiency for each filtration class (G1, G2, G3, G4, M5, M6, F7, F8 and F9) as well as a minimum filtration efficiency starting from filtration class F7. Said filters comply with the EN1882 classification standard, replaced by the international standard ISO 16890:2016.

In addition to these filters, classified by the European standard EN 779, which prevent the passage of particles of different sizes, air purification devices also use filters that work with activated carbon, but the objective of which is to filter the captured air, by passing said air through chemical adsorbents, which are capable of eliminating the gases in the air with efficiencies of up to 99%.

Filters of this type are configured to absorb complex gas molecules while the simpler molecules are transformed in a chemical reaction, which by means of the same activated carbon is impregnated with potassium permanganate.

These types of filters are used because, in addition to the suspended particles, the air can also be altered with a high presence of polluting gases such as volatile organic compounds (VOC) which are chemical substances that contain carbon and that easily are converted into vapours or gases, which affect people's health.

Other polluting gases very present in the urban and industrial environment are those considered greenhouse gases (GHG), such as carbon dioxide (CO₂), nitrogen oxides (NO_{X}), which is applied to various compounds formed by the combination of oxygen and nitrogen, ozone (O₃) or chlorofluorocarbons (CFCs).

Polluting gases of this type directly affect people's health, and air cleaning devices use different technologies to reduce the concentration thereof, such as photocatalytic reduction.

Photocatalysis is one of the variants of advanced oxidation processes that combines ultraviolet light and oxidation by means of a catalyst in such a way that polluting gases, odours and microorganisms are eliminated, improving air quality with very efficient energy consumption. This technology is used in purification devices to decontaminate the air of harmful substances such as NO_{X}, SO_{X} or VOCs among others.

PCT application with publication no. WO 2020/058547 A1 relates to an integrated autonomous air purification device comprising several groups of air filtering elements through which upward air is filtered, such that the air enters the inside of the device through a lower inlet and exits to the outside through an upper nozzle. The device includes suction means to suck a polluted air flow from the outside, conduct it inside the device through all the groups of filtering elements, in order to finally expel the clean air flow to the outside through the upper nozzle, wherein obviously the polluting elements have obviously been retained in the different groups of filtering elements.

The described filtration systems are not designed to be able to simultaneously retain particulate pollutants and remove bacteria and other pathogens such as viruses.

### Description of the invention

In order to achieve the objectives and prevent the drawbacks mentioned in the preceding sections, the invention proposes an air purification device comprising a case having a lower air inlet and an upper air outlet, wherein the case delimits an inner space in which there is located at least one filtering system configured so that an upward air flow can circulate through same for filtration thereof entering through the lower inlet and exiting through the upper outlet with the help of a fan device with an upward suction effect.

Said fan device is located above at least the filtering system within the inner space delimited by the case.

There is located within the inner space of the case at least a first filtering system comprising an M6 filter according to standard EN1822, a second filtering system including UV-C lamps, and a third filtering system, wherein the second filtering system is situated between the first filtering system and the third filtering system, wherein the ultraviolet light UV-C from the lamps sterilizes the air flow crossing through the second filtering system, and wherein the ultraviolet light UV-C from the lamps has a powerful destructive effect on the DNA chains of microorganisms or cells retained in the first filtering system and the third filtering system and also on those of microorganisms or cells in the space delimited between the first filtering system and the third filtering system.

The third filtering system is a filter selected from a HEPA H14 filter according to standard EN1822, and a ULPA U15 or U16 or U17 filter according to standard EN1822, wherein the HEPA H14 filter ensures a retention of 99.97% of particles of up to 0.12 microns, while the ULPA U17 filter ensures a retention of particles of up to 0.01 microns.

The device of the invention comprises an electronic safety device associated with a door providing access to the UV-C lamps, wherein the electronic safety device includes a switch associated with the door, such that when said door is opened, the current circulation powering the UV-C lamps is interrupted and when said door is closed, the electric current powering the UV-C lamps is reactivated.

The device of the invention comprises an electronic device associated with the UV-C lamps and the fan device, wherein to prevent the lamps from overheating, they will only be switched on while the fan device that generates the air flow which cools the UV-C lamps is activated.

The inner space delimited by the case comprises a lower chamber communicating with the outside through the lower inlet, an upper chamber into which the upper outlet flows, and a main chamber delimited between the lower chamber and the upper chamber.

The three filtering systems are located inside the main chamber, wherein a fan device for forcing air circulation with the suction effect through the inside of the case from the lower inlet to the upper outlet is located inside the upper chamber of the case.

Next, to help better understand this specification and as an integral part thereof, a series of figures is attached in which the object of the invention is depicted in an illustrative and non-limiting manner.

### Brief description of the figures

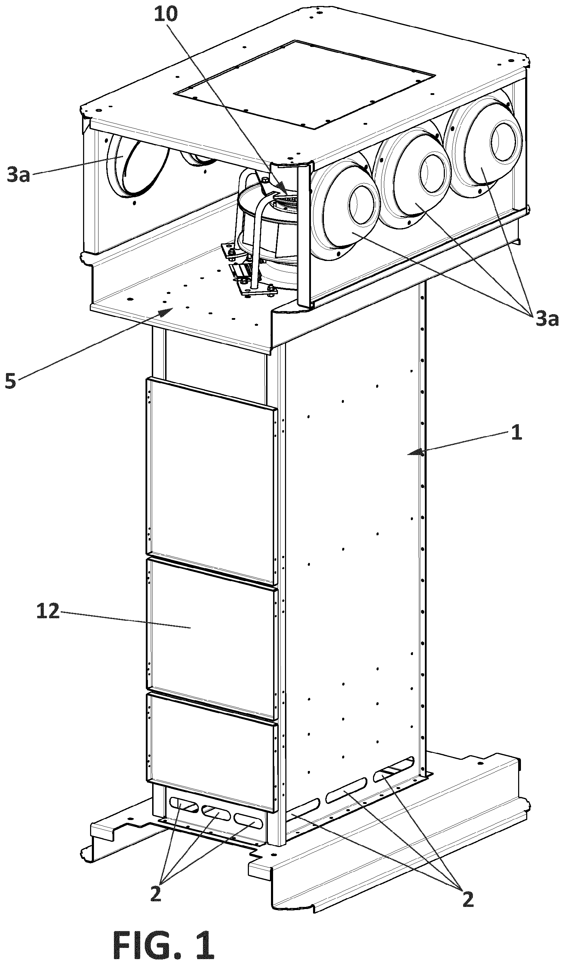

**Figure 1** shows a perspective view of the air purification device object of the invention.

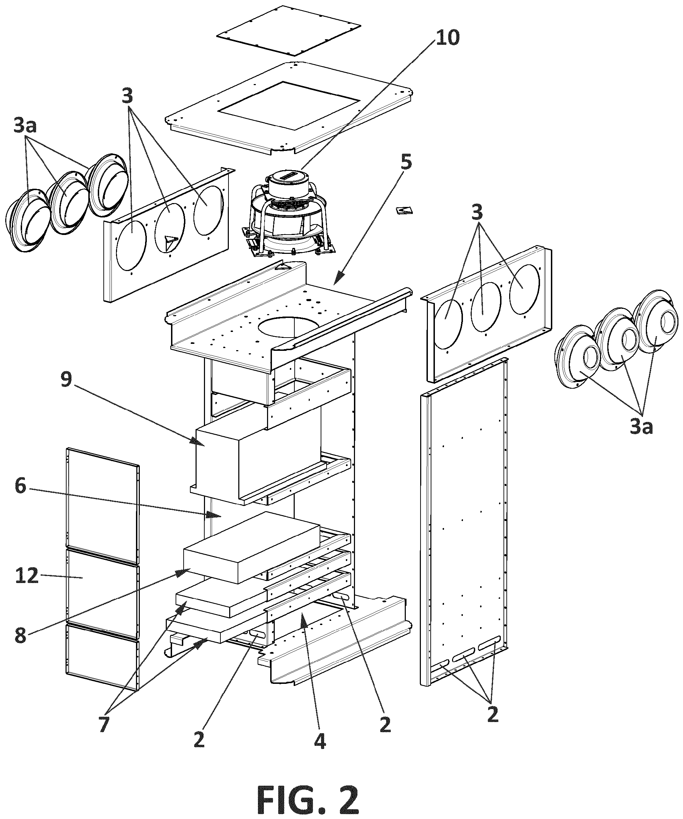

**Figure 2** shows an exploded view of the device of the invention.

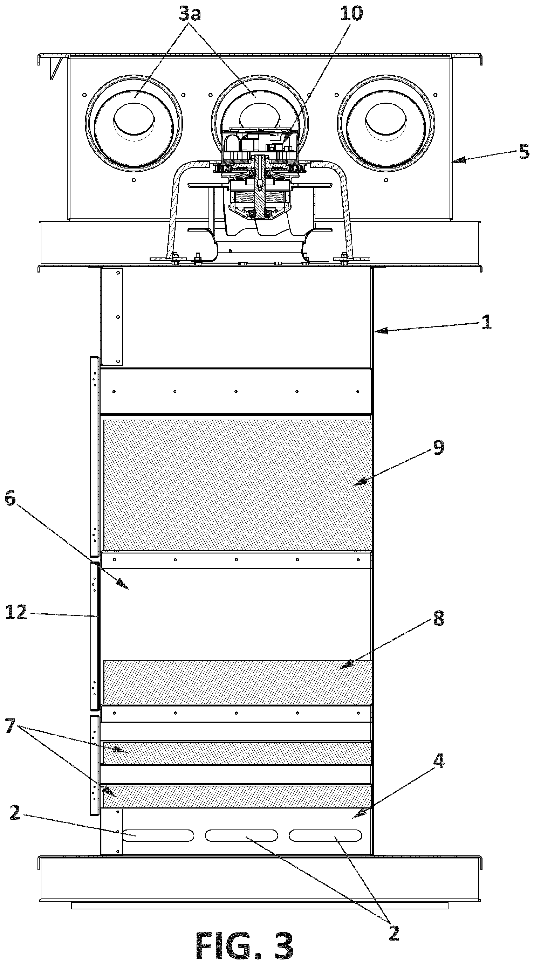

**Figure 3** shows a sectioned elevational view of the air purification device.

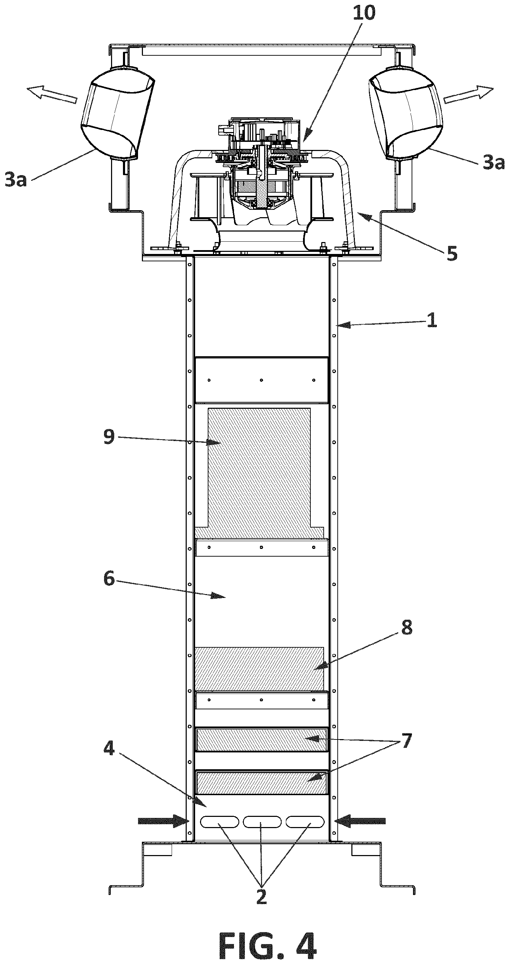

**Figure 4** shows a sectioned profile view of the air purification device.
**Figure 5** shows a view of an electronic safety circuit associated with an access door to the inner space of the air purification device.

### Description of an exemplary embodiment of the invention

Taking into consideration the numbering used in the figures, the air purification device comprises a case 1 having a lower air inlet 2 and an upper air outlet 3 where nozzles 3a are connected.

A lower chamber 4 communicating with the outside through the lower inlet 2, an upper chamber 5 into which the upper outlet 3 flows, and a main chamber 6 delimited between the lower chamber 4 and the upper chamber 5 are configured inside the case 1.

A first filtering system 7, a second filtering system 8 including UV-C lamps 11, and a third filtering system 9 (H14) are located inside the main chamber 6 of the case 1.

In contrast, there is located inside the upper chamber 5 of the case 1 a fan device 10 for forcing air circulation with a suction effect through the inside of the case 1 from the lower inlet 2 to the upper outlet 3, with the upward air flow obviously passing inside the case 1 through the three filtering systems having the function of retaining the harmful elements in the air, both polluting elements and various pathogens such as bacteria and viruses such as the "Covid 19" virus.

Thus, the air is captured at the lower part and passes upwardly through the different filtering systems that capture and/or destroy the polluting elements. Finally, the cleaned or purified air is released to the outside through the upper outlet 3 situated at the upper part of the case 1.

As the air flow travels upwardly inside the case 1, in a first phase the air is filtered through the first filtering system 7; in a second phase the air is filtered through the second filtering system 8, and in a third phase the air flow is filtered through the third filtering system 9, in order to finally reach the upper chamber and is expelled to the outside through the upper outlet 3.

The first filtering system 7 comprises an M6 filter according to standard EN1822 which mainly retains elements of a large size in a first phase, although other elements of a smaller size may also remain in its structure.

The third filtering system 9 comprises a filter selected from a HEPA filter H14 according to standard EN1822, and a ULPA U15 or U16 or U17 filter according to standard EN1822.

The HEPA H14 filter ensures a retention of 99.97% of particles of up to 0.12 microns, while the ULPA U17 filter ensures a retention of particles of up to 0.01 microns.

In one embodiment, the device of the invention would incorporate as standard a filtering system 9 with an H14 filter, which are the same as those used in negative pressure rooms for infectious diseases (SARS, EBOLA, COVID-19, etc.) in hospitals.

The second filtering system 8 comprises a 245-nanometer UV-C stage with TiO2 cells. This UV-C light has a powerful destructive effect on the DNA chains of any microorganism or cell.

The second filtering system 8 with the UV-C lamps 11 is placed before the third filtering system 9 with the H14 filter for two fundamental reasons, which can be considered the most original and advantageous of the invention:
- First, they disable all viruses that have been retained in the third filtering system with the H14 filter, as well as viruses that have also been retained in the first filtering system 7 located below the second filtering system 8.
- Second, the UV-C ultraviolet light sterilizes the air passing through the lamps 11 of the second filtering system 8.

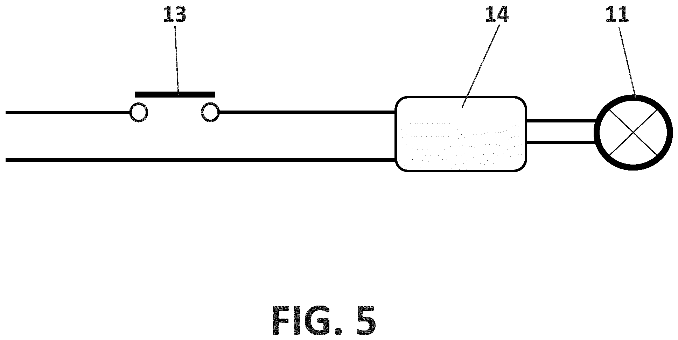

Since ultraviolet light UV-C should not be irradiated or projected directly on a person's skin or eyes, the device of the invention incorporates an electronic safety device (Figure 5) which disconnects the lamps 11 when a door 12 providing access to said lamps 11 emitting the ultraviolet light UV-C is opened.

Basically, it is a switch 13 associated with the door 12, such that when the door 12 is opened, the current circulation powering the UV-C lamps 11 is interrupted and when the door 12 is closed, the electric current powering the UV-C lamps 11 is reactivated.

The electronic safety device further includes an electronic ballast 14 which has the function of keeping the UV-C lamps 11 stable and limiting their intensity.

To prevent the lamps 11 from overheating, they will only be switched on when there is an air flow that allows their cooling, such that to prevent said overheating an electronic device which disconnects the lamps 11 when the fan device is stopped has been envisaged.

Therefore, first, it is emphasised that with the filtering device of the invention, the combination of the third filtering system 9 with the H14 filter and the second filtering system 8 with the UV-C light deactivates the coronaviruses retained in the first filtering system 7 and the third filtering system 9 located, respectively, below and above the UV-C light lamps 11 included in the second filtering system 8.

Second, it is emphasised that with this effect described in the preceding paragraph, there is the possibility of continuously using the device of the invention to filter gases as well.

Third, the strategic location of the UV-C lamps 11 is highlighted, as they are located between the two filtering systems: the first filtering system 7 and the third filtering system 9, and not at the air outlet or inlet.

Taking into consideration PCT patent with publication no. WO 2020/058547A1 which also refers to an air purification device, its structure could easily be adapted to achieve the advantages obtained in the invention.

To that end, said PCT patent could replace the activated carbon filter with a HEPA H14 filter according to standard EN1822 or a ULPA U15 or U16 or U17 filter according to standard EN1822, and replace the UV-A part with TiO2 cells with a 245-nanometer UV-C stage.

## Claims

1. **Air purification device,** comprising a case (1) having a lower air inlet (2) and an upper air outlet (3), wherein the case (1) delimits an inner space in which there is located at least one filtering system configured so that an upward air flow can circulate through same for filtration thereof entering through the lower inlet (2) and exiting through the upper outlet (3) with the help of a fan device (10) with an upward suction effect, wherein said fan device (10) is located above the filtering system within the inner space delimited by the case (1), **characterised in that**:
- there is located within the inner space of the case (1) at least a first filtering system (7) comprising an M6 filter according to standard EN1822, a second filtering system (8) including UV-C lamps (11), and a third filtering system (9), wherein the second filtering system (8) is situated between the first filtering system (7) and the third filtering system (9), wherein the ultraviolet light UV-C from the lamps (11) sterilizes the air flow crossing through the second filtering system (8), and wherein the ultraviolet light UV-C from the lamps (11) has a powerful destructive effect on the DNA chains of microorganisms or cells retained in the first filtering system (7) and the third filtering system (9) and also on those of microorganisms or cells in the space delimited between the first filtering system (7) and the third filtering system (9);
- the third filtering system (9) is a filter selected from a HEPA H14 filter according to standard EN1822, and a ULPA U15 or U16 or U17 filter according to standard EN1822, wherein the HEPA H14 filter ensures a retention of 99.97% of particles of up to 0.12 microns, while the ULPA U17 filter ensures a retention of particles of up to 0.01 microns.

2. **Air purification device** according to claim 1, **characterised in that** it comprises an electronic safety device associated with a door (12) providing access to the UV-C lamps (11), wherein the electronic safety device includes a switch (13) associated with the door (12), such that when said door (12) is opened, the current circulation powering the UV-C lamps (11) is interrupted and when said door (12) is closed, the electric current powering the UV-C lamps (11) is reactivated.

3. **Air purification device** according to any one of the preceding claims, **characterised in that** it comprises an electronic device associated with the UV-C lamps (11) and the fan device (10), wherein to prevent the lamps (11) from overheating, they will only be switched on while the fan device (10) that generates the air flow which cools the UV-C lamps (11) is activated.

4. **Air purification device** according to any one of the preceding claims, **characterised in that** the inner space delimited by the case (1) comprises a lower chamber (4) communicating with the outside through the lower inlet (2), an upper chamber (5) into which the upper outlet (3) flows, and a main chamber (6) delimited between the lower chamber (4) and the upper chamber (5), wherein the three filtering systems (7, 8, 9) are located inside the main chamber (6), and wherein a fan device (10) for forcing air circulation with the suction effect through the inside of the case (1) from the lower inlet (2) to the upper outlet (3) is located inside the upper chamber (5) of the case (1).
